# EUROPEAN PATENT SPECIFICATION

(11) **EP 1 084 823 B1**
(45) Date of publication and mention of the grant of the patent: **09.03.2005**
(21) Application number: 00500183.9
(22) Date of filing: 08.08.2000
(51) Int. Cl.: B32B 15/08, B32B 27/36, A61B 19/08

(54) **Laminate for medical surgery applications**
Mehrschichtiger, medizinischer Gegenstand für die Chirurgie
Article médical stratifié à usage chirurgical

(30) Priority: 25.08.1999 ES 9901942
(43) Date of publication of application: 21.03.2001
(73) Proprietor: Thermical Medic, S.L., 08960 Sant Just Desvern (ES)
(72) Inventor: Domenech Rabasa, Jordi, 08960 Sant Just Desvern (ES)
(74) Representative: SUGRANES - VERDONCES - FERREGÜELA

(56) References cited:
- EP-A- 0 365 326
- DE-A- 3 113 888
- US-A- 4 360 984

## Description

The present invention refers to a laminate whose structure and composition give it remarkable qualities which are particularly suitable for medical surgery uses, as well as in other applications which require protective physical behavior for people or things protected by this material.

Among the considerable range of possibilities applicable to this material, it is possible to highlight its great capacity for clinical use, as is also the case with another material described by the same applicant in the document of Spanish patent U 9402540 (publication ES-A-U 1029090), the material of the present invention being of a new and distinct configuration and reaching new fields of application. In the same way as was indicated in the Spanish patent document U 9402540, the material in accordance with the present invention also has excellent qualities for clinical use, both for the manufacture of items for clinical use and especially for the care of post-operative patients since its insulating qualities from both heat and electricity, anti-glare and coloring possibilities facilitate the making of that type of item.

Moreover, the applications of this material are enormous, centering on their high degree of usefulness in the field of medical surgery accessories, in addition to the food preservation field and, above all, for the manufacture and production of safety items du to its insulating capacity from both heat and electricity.

It should also be taken into account that the laminate presented herein, is not only of use for the protection and manufacture of objects or items for the applications which have been indicated, as if it were fabrics, but that it can also be used for covering parts or for the protection of other materials.

The combination of several superimposed layers with specially chosen characteristics gives rise to this new laminate which has the following main features:
- It is dielectric. This means that it is possible to say that the electrical conductivity of same is almost non-existent and therefore its application in the protection of clinical patients, particularly as a safety factor when there are surgical instruments nearby which work by electricity.
- It is isothermal. This quality gives it its great applicability in the making up of or use as a clinical blanket or similar.
- It is waterproof. Thanks to the qualities of the components of same, the material in accordance with the invention is waterproof and absorbent at the same time.
- It is aseptic. That is, it rejects the propagation of infections, particularly in the clinical field, because it is highly waterproof, which does not facilitate perspiration. It is also worth mentioning that it has other qualities of lesser importance such as its softness, it is not noisy when handled, the lack of perspiration for alcoholic and detergent products, so it is an ideal blanket, and it is also resistent to physical deterioration.

In accordance with the invention, the laminate is composed of four superimposed layers of a different nature, according to the following pattern:

The first layer is a very thin film of colored and/or transparent, non-skid and shaded polyethylene; it is followed by a layer of metallized polyester, the metallized side of which makes contact with the previously mentioned polyethylene layer. Next, there is a layer of polyurethane foam and is followed by the final layer which is a woven or non-woven fabric, in the form of a thin film. The end result is a resistant metallized sheet which on touch appears to be made up of a single layer or body.

The laminate for medical surgery applications in accordance with the present invention is characterized in that its is composed of a colored and/or transparent polyethylene non-skid and shaded first layer; an embedded metallized polyester second layer firmly joined to the first layer by means of a suitable adhesive specially by its metallized side; a third layer of polyurethane foam which is adhered by applying heat and pressure to the metallized polyester layer by the opposite side to the metallized side; and a final outer layer of woven or non-woven fabric firmly joined to the previous polyurethane foam layer, the whole laminate having the look of a flexible metallized sheet on one of its sides.

### PECULIARITIES OF THE LAMINATE IN ACCORDANCE WITH THE INVENTION:

a) The colored or transparent, non-skid and shaded polyethylene normally has a thickness of between 10 and 20 microns, preferably 15 microns.
b) The metallized polyester layer normally has a thickness of between 10 and 15 microns, preferably 12 microns.
c) The polyurethane foam layer normally has a thickness of between 1 mm and 3 mm.
d) Normally, the first polyethylene layer is matt since for clinical purposes it is not advisable for it to be dazzling so that it does not hinder medical work.
e) It is provided that the polyethylene be colored, preferably green which is the one commonly used in clinic. Above all, in the sterilized areas of operating theaters.
f) The woven or non-woven fabric which can form the final layer is preferably made of nylon or fine netting.
g) The polyurethane foam sheet adds an absorption capacity in situ to the result which increases the quality of the whole as it acts as a blotter for fluids such as blood, preventing them from running through to the outside. In this way, in a surgical application, the patient is free from stains or other impregnations.

For a better understanding of the invention, the sheet of drawings accompanying the present description shows by way of non-restrictive example, a large scale diagrammatic cross-section representation of the laminate for surgical applications to which it refers.
Figure 1 shows a partial portion of the laminate seen in cross-section, in which the structure solely comprises two of the elements characterizing it.
Figure 2 is the same partial portion of the laminate shown in Figure 1, seen in perspective.
Figure 3 also corresponds to a portion of the laminate in accordance with the invention seen in cross-section, in this case comprising all the elements with respect to the example shown in Figures 1 and 2.
Figure 4 is a view in which the diagram of the laminate shown in Figure 3 is drawn in perspective.

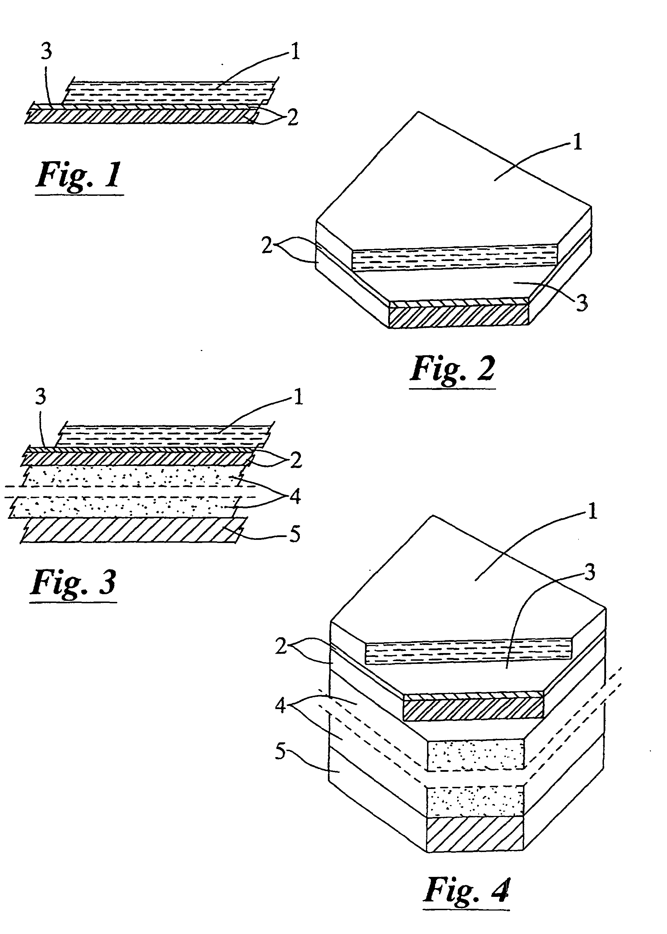

In accordance with Figures 1 and 2 the following can be seen: the polyethylene colored and/or transparent, anti-skid and shaded layer (1) joined to which is the metallized polyester layer (2), the metallized side (3) being situated in contact with the polythene layer (1) previously mentioned.

The invention which is shown in Figures 3 and 4, comprises, in addition to layers (1) and (2), a layer of polyurethane foam (4) which is found next to layer (2), followed by a layer formed by a film of woven or non-woven fabric (5).

The joining of the aforesaid layers give rise to the formation of the laminate in accordance with the invention, whose features have been defined earlier.

## Claims

1. Laminate for medical surgery applications **characterized in that** its is composed of a colored and/or transparent polyethylene non- skid and shaded first layer (1) ; an embedded metallized polyester second layer (2) firmly joined to the first layer (1) by means of a suitable adhesive specially by its metallized side (3); a third layer (4) of polyurethane foam which is adhered by applying heat and pressure to the metallized polyester layer (2) by the opposite side to the metallized side (3); and a final outer layer (5) of woven or non-woven fabric firmly joined to the previous polyurethane foam layer, the whole laminate having the look of a flexible metallized sheet on one of its sides.

2. Laminate for medical surgery applications, in accordance with claim 1, **characterized in that** the anti-skid and shaded polyethylene layer (1) has a thickness of between 10 and 20 microns.

3. Laminate for medical surgery applications, in accordance with any one of the previous claims, **characterized in that** the metallized polyester layer (2) has a thickness of between 10 and 15 microns.

4. Laminate for medical surgery applications, in accordance with 1), **characterized in that** the polyurethane foam layer (4) has a thickness of between 1 mm and 3 mm.

## Patentansprüche

1. Laminat für chirurgische Anwendungen, **dadurch gekennzeichnet, daß** es zusammengesetzt ist aus einer rutschfesten und nuancierten ersten Schicht (1) aus farbigem und/oder transparentem Polyethylen; einer eingebetteten zweiten Schicht (2) aus metallisiertem Polyester, die fest verbunden ist mit der ersten Schicht (1) mittels eines geeigneten Haftmittels, und zwar speziell über ihre metallisierte Seite (3); einer dritten Schicht (4) aus Polyurethanschaumstoff, die angehaftet wird durch Ausüben von Wärme und Druck auf die Schicht (2) aus metallisiertem Polyester auf der zu der metallisierten Seite (3) entgegengesetzten Seite; und einer abschließenden äußeren Schicht (5) aus gewebtem oder nicht gewebtem Stoff, die fest verbunden ist mit der vorhergehenden Schicht aus Polyurethanschaumstoff, wobei das gesamte Laminat an einer seiner Seiten das Aussehen einer schmiegsamen, metallisierten Folie hat.

2. Laminat für chirurgische Anwendungen nach Anspruch 1, **dadurch gekennzeichnet, daß** die rutschfeste und nuancierte Polyethylenschicht (1) eine Dicke von zwischen 10 und 20 Mikrometern hat.

3. Laminat für chirurgische Anwendungen nach einem der vorstehenden Ansprüche, **dadurch gekennzeichnet, daß** die metallisierte Polyesterschicht (2) eine Dicke von zwischen 10 und 15 Mikrometern hat.

4. Laminat für chirurgische Anwendungen nach Anspruch 1, **dadurch gekennzeichnet, daß** die Schicht (4) aus Polyurethanschaumstoff eine Dicke von zwischen 1 mm und 3 mm hat.

## Revendications

1. Stratifié pour des applications médicales en chirurgie, **caractérisé en ce qu'**il est composé d'une première couche (1) de polyéthylène coloré et/ou transparent anti-dérapant et ombré; d'une deuxième couche (2) en polyester métallisée incluse fermement fixée à la première couche (1) au moyen d'un adhésif adapté spécialement par sa face métallisée (3); une troisième couche (4) en mousse de polyuréthane qui est fixée par application de chaleur et de pression à la couche (2) en polyester métallisé sur la face opposée à la face (3) métallisée; et une couche externe finale (5) en étoffe tissée ou non tissée fermement fixée à la couche précédente en mousse de polyuréthane, l'ensemble du stratifié ayant l'apparence d'une feuille métallisée souple par l'une de ses faces.

2. Stratifié pour des applications médicales en chirurgie, conformément à la revendication 1, **caractérisé en ce que** la couche (1) de polyéthylène anti-dérapant et ombré a une épaisseur entre 10 et 20 microns.

3. Stratifié pour des applications médicales en chirurgie, conformément à l'une quelconque des revendications précédentes, **caractérisé en ce que** la couche (2) en polyester métallisé a une épaisseur entre 10 et 15 microns.

4. Stratifié pour des applications médicales en chirurgie, conformément à la revendication 1, **caractérisé en ce que** la couche (4) en mousse de polyuréthane a une épaisseur entre 1 mm et 3 mm.
